# EUROPEAN PATENT APPLICATION

(11) **EP 3 872 493 A1**
(43) Date of publication of application: **01.09.2021**
(21) Application number: 19875163.8
(22) Date of filing: 24.10.2019
(51) Int. Cl.: G01N 33/68, G01N 33/50, G01N 33/53, C07K 14/47

(54) **TEST METHOD FOR MAMMALIAN OLFACTORY MUCOSA OR OLFACTORY FLUID**

(30) Priority: 24.10.2018 JP 2018200100
(71) Applicant: AJINOMOTO CO., INC., Chuo-ku Tokyo 104-8315 (JP)
(72) Inventor: IJICHI, Chiori, Kawasaki-shi, Kanagawa 210-8681 (JP); KONDO, Kenji, Tokyo 113-8654 (JP); SHIRASAWA, Ayaka, Kawasaki-shi, Kanagawa 210-8681 (JP); NAKATA, Kunio, Kawasaki-shi, Kanagawa 210-8681 (JP); SHIMBO, Kazutaka, Kawasaki-shi, Kanagawa 210-8681 (JP); MARUYAMA, Yutaka, Kawasaki-shi, Kanagawa 210-8681 (JP); KAWATO, Yayoi, Kawasaki-shi, Kanagawa 210-8681 (JP)
(74) Representative: Hoffmann Eitle
(86) International application number: PCT/JP2019/041786
(87) International publication number: WO 2020/085458

(57) **Abstract**

The present invention provides a technique that can non-subjectively and objectively diagnose olfactory sense and is easily applied to clinical practice. More specifically, the present invention provides a test method for olfactory mucosa or olfactory mucus in a mammal comprising measuring an amount of lipocalin 15 in an olfactory mucosa sample or an olfactory mucus sample taken from the mammal, and a test reagent for the olfactory mucosa or the olfactory mucus in the mammal comprising a detection means for lipocalin 15.

## Description

### TECHNICAL FIELD

The present invention relates to a test method and a test reagent for olfactory mucosa or olfactory mucus.

### BACKGROUND ART

Currently in clinical practice, olfactory disorders are diagnosed by determining whether a patient can detect the presence of odor and identify a type of the odor when the patient smells an odorous substance. For diagnosis of the olfactory disorder, a plurality of test kits is available inside and outside of the country, but all of them are subjective test methods based on a patient's response. For non-subjective/objective methods, only methods at laboratory levels such as measurement of an electro-olfactogram when olfactory stimulus is given are available and there is no technique available for clinical practice.

### PRIOR ART REFERENCES

### [NON-PATENT LITERATURES]

Non-patent Literature 1: Takaki Miwa, "Current status and future prospects of olfactory test", Oto-Rhino-Laryngology, Tokyo, 54:2; 70-79, 2011.

### DISCLOSURE OF INVENTION

### PROBLEM TO BE SOLVED BY THE INVENTION

It is an object of the present invention to develop a technique that can non-subjectively and objectively diagnose olfactory sense and is easily applied to clinical practice.

### MEANS FOR SOLVING PROBLEM

As a result of an extensive study, the present inventors have found that lipocalin 15 is present as a secreted protein specifically produced in olfactory mucosa and secreted into olfactory mucus. Based on such a finding in consideration of a fact that the olfactory mucosa can closely associated with the olfactory sense, it is conceivable that the olfactory sense can be tested by measuring an amount of the lipocalin 15 in an olfactory mucosa sample or an olfactory mucus sample.

That is, the present inventors have successfully arrived at the following inventions and completed the present invention.
[1] A test method for olfactory mucosa or olfactory mucus in a mammal, comprising measuring an amount of lipocalin 15 in an olfactory mucosa sample or an olfactory mucus sample taken from the mammal.
[2] The test method of [1], wherein the amount of lipocalin 15 as a protein is measured using an antibody against lipocalin 15.
[3] The test method of [1] or [2], wherein the amount of lipocalin 15 as a protein is measured in the olfactory mucus sample taken from the mammal.
[4] The test method of any of [1] to [3], wherein the mammal is a human.
[5] A test reagent for olfactory mucosa or olfactory mucus in a mammal, comprising a detection means for lipocalin 15.
[6] The test reagent of [5], wherein the detection means is an antibody.

### EFFECT OF THE INVENTION

The method of the present invention can non-subjectively and objectively diagnose the olfactory mucosa, or a function (e.g., olfactory sense) or a dysfunction (e.g., olfactory disorder) associated with the olfactory mucosa by measuring an amount of lipocalin 15.

In terms of measurement of the amount of lipocalin 15, the method of the present invention is also a convenient technique easily applied to the clinical practice rather than a method confined to a laboratory level such as measurement of electro-olfactogram.

Furthermore, the method of the present invention has an advantage of low invasiveness when the olfactory mucus taken from the mammal is used as the sample.

The test reagent can be utilized suitably for practicing the present invention.

### BRIEF DESCRIPTION OF DRAWINGS

Fig. 1 shows expression of His-hLCN15 in *E. coli.* Lane 1: protein markers (Nacalai Tesque); lane 2: microbial cells expressing His-hLCN15.
Fig. 2 shows analysis for purification degree and a molecular mass of hLCN15. Lane 1: protein markers (Nacalai Tesque); lane 2: purified hLCN15.
Fig. 3 shows detection of hLCN15 in olfactory mucus samples and no detection of hLCN15 in respiratory epithelial mucus samples.
Fig. 4 shows immunostaining of human nose sections using hLCN15 antibody. (a) Human nose large section stained with H/E; (b) olfactory mucosa part just below olfactory bulb stained with hLCN15 antibody; (c) middle nasal concha stained with hLCN15 antibody; and (d) inferior nasal concha stained with hLCN15 antibody.
Fig. 5 shows immunostaining of human nose sections using OMP antibody (olfactory neural cell marker). (a) Olfactory mucosa part just below olfactory bulb stained with OMP antibody (hOMP); and (b) magnified view.
Fig. 6 shows immunostaining of human nose sections using hLCN15 antibody (magnified view of Fig. 4(b)). (a) Olfactory mucosa just below olfactory bulb stained with hLCN15 antibody; and (b) magnified view (serous gland).
Fig. 7 shows results of immunostaining of a human specimen (Individual 1: specimen with few olfactory nerves) stained with antibodies against olfactory nerve (PGP9.5), support cell (CK18), and LCN15, respectively.
Fig. 8 shows results of immunostaining of a human specimen (Individual 2: specimen with abundant olfactory nerves) stained with antibodies against olfactory nerve (PGP9.5), support cell (CK18), and LCN15, respectively.
Fig. 9 shows one example of a standard curve for quantification of hLCN15 in human olfactory mucus using the hLCN15 antibody.
Fig. 10 shows comparison of amounts of the LCN15 protein between a normal olfactory sense group and an impaired olfactory sense group. The amount of the LCN15 protein in the impaired olfactory sense group was significantly smaller than that in the normal olfactory sense group (Welch t-test, p=0.009).

### EMBODIMENTS FOR CARRYING OUT THE INVENTION

The present invention provides a test method for olfactory mucosa or olfactory mucus in a mammal, comprising measuring an amount of lipocalin 15 in an olfactory mucosa sample or an olfactory mucus sample taken from the mammal.

Lipocalin 15 is a secreted protein produced in the olfactory mucosa and secreted into the olfactory mucus. As lipocalin 15, those derived from various mammalian species as described later can be utilized, but in terms of clinical practice, human lipocalin 15 is preferred. It is known that human lipocalin 15 has the amino acid sequence of SEQ ID NO:1.

Examples of the mammals include primates (e.g., humans, monkeys, chimpanzees), rodents (e.g., mice, rats, hamsters, guinea pigs, rabbits), farm animals and working animals (e.g., cattle, swine, sheep, goats, horses). Preferably, the mammal is a human in terms of clinical practice.

An olfactory mucosa sample or an olfactory mucus sample can be taken from a mammal by any method. For example, the olfactory mucosa sample can be taken from the mammal by biopsy. Also, the olfactory mucus sample can be taken from the mammal by washing an olfactory region at an upper nasal cavity with a solution such as saline and collecting the washing solution. In terms of noninvasiveness, it is preferred to collect the olfactory mucus sample.

An amount of lipocalin 15 can be measured in an olfactory mucosa sample or an olfactory mucus sample by any method. For example, the amount of lipocalin 15 in the olfactory mucosa sample where lipocalin 15 is expressed can be measured by measuring an amount of mRNA or a protein. Also, the amount of lipocalin 15 in the olfactory mucus sample where lipocalin 15 is secreted can be measured by measuring the amount of the protein. Methods of measuring the amount of mRNA include, for example, a hybridization method using a probe, a gene amplification method using primers (e.g., 2, 3 or 4 primers), and a mass spectrometry method. The measurement of an mRNA amount may be combined with reverse transcription of mRNA. Methods of measuring the amount of the protein include, for example, an immunoassay method using antibodies, an assay method using an affinity substance (e.g., an aptamer) other than an antibody, and a mass spectrometry method. The immunoassay methods include a chemiluminescence immunoassay (CLIA) [e.g., chemiluminescence enzyme immunoassay (CLEIA)], a turbidimetric immunoassay (TIA), an enzyme immunoassay (EIA) [e.g., direct competitive ELISA, indirect competitive ELISA, and sandwich ELISA], a radioimmunoassay (RIA), a latex aggregation assay, a fluorescence immunoassay (FIA), and an immunochromatography method, a western blotting method, and an immunostaining method.

Preferably, in terms of noninvasiveness, the amount of lipocalin 15 can be measured by an immunoassay using antibodies and using an olfactory mucus sample as an analysis subject.

Antibodies used in the immunoassay may be polyclonal antibodies or monoclonal antibodies. An antibody may be any isotype of immunoglobulins (e.g., IgG, IgM, IgA, IgD, IgE, IgY). An antibody may be a full length antibody or a fragment thereof. A fragment of an antibody includes, for example, F(ab')2, Fab', Fab and Fv. In the present invention, not only one antibody but also two or more (e.g., two, three types) antibodies can be used depending on a type of the immunoassay to be used.

An antibody may be labelled with a labelling substance. Examples of labelling substances include enzymes (e.g., peroxidase, alkaline phosphatase, luciferase), affinity substances (e.g., streptavidin, biotin), fluorescent substances and proteins (e.g., fluoresceine, rhodamine, green fluorescent protein), luminescent or light-absorption substances (e.g., luciferin), and radioactive substances (e.g., ³H, ¹⁴C, ³²P, ³⁵S, ¹²⁵I).

Examples of immunoassay methods include a direct competitive method, an indirect competitive method and a sandwich method. Also, examples of immunoassay methods include an enzyme immunoassay (EIA) (e.g., direct competitive ELISA, indirect competitive ELISA, and sandwich ELISA), a radioimmunoassay (RIA), a fluorescence immunoassay (FIA), a chemiluminescence immunoassay (CLIA), an immunochromatography method, a latex aggregation assay, and a western blotting method.

In the method of the present invention, a function (e.g., olfactory sense) or a dysfunction (e.g., olfactory disorder) associated with the olfactory mucosa may be evaluated based on the amount of measured lipocalin 15. Since the olfactory mucosa can closely be associated with the olfactory sense (e.g., Yamagishi et al., Practica Oto-Rhino-Laryngologica, Vol. 83, No. 3 pages 383-890; Miani et al., Eur. Arch. Otorhinolaryngol, 2003, vol. 260, p.529-535; Netzer et al., J Otolaryngol., 2002, vol. 31(1), p.9-12; Kondo et al., Cell Tissue Res, 2009, vol. 335(3), p.489-503); Hurtt et al., Toxicology and Applied Pharmacology, 1988, vol. 94(2), p.311-328; Kim et al., Otolaryngol Head Neck Surg., 2010, vol. 143(6), p.837-42; Kikuta et al., Sci Rep., 2016, vol. 6, p.35361), the amount of lipocalin 15 specifically produced by the olfactory mucosa can become an indicator for an amount of normal olfactory mucosa and thus for the olfactory sense. For example, when the olfactory mucosa is in an abnormal state (e.g., damage or disorder), amounts of lipocalin 15 mRNA and protein in the olfactory mucosa as well as an amount of the lipocalin 15 protein secreted into the olfactory mucus can be reduced. Therefore, lipocalin 15 can be useful as the indicator as described above.

The evaluation as above can be performed, for example, by comparing the amount of the measured lipocalin 15 with a standard value such as a cutoff value. Calculation of the standard value such as the cutoff value is well-known in the art. For example, amounts of lipocalin 15 are analyzed in a normal group and an abnormal group, diagnostic sensitivity and diagnostic specificity are obtained at predetermined amounts of lipocalin 15, and an ROC (receiver operating characteristic) curve is prepared based on these values. And, an amount of lipocalin 15 that maximizes the diagnostic sensitivity and the diagnostic specificity is obtained, and this value can be used as the cutoff value. Also, diagnostic efficiency (percentage of sum of normal proper diagnosis cases and abnormal proper diagnosis cases based on total cases) at any amount of lipocalin 15 is obtained, and the amount of lipocalin 15 by which the highest diagnostic efficiency is calculated can also be used as the cutoff value. Alternatively, in order to exclude as much as possible that a subject belonging to the abnormal group is wrongly evaluated as one belonging to the normal group (false positive), the cutoff value may be determined with more emphasis on the diagnostic specificity.

The present invention also provides a test reagent for olfactory mucosa or olfactory mucus in a mammal, comprising a detection means for lipocalin 15.

Details of lipocalin 15 and the mammal are the same as those described above.

The detection means for lipocalin 15 is a detection means for mRNA or a protein.

Examples of the detection means for mRNA include primers (e.g., two or more primers) and nucleic acid probes depending on a gene amplification method to be used. The nucleic acid probe can be used in a free form or an immobilized form to a solid phase. Examples of the solid phase include a support such as an array, a membrane (e.g., nitrocellulose membrane, filter) and a column; and a container such as a plate (e.g., multi-well plate) and a tube.

Examples of the detection means for the protein include antibodies and aptamers. These detection means may be labelled with the labelling substance as described above. When these detection means are not labeled with the labelling substance, the test reagent of the present invention may be configured to comprise the labelling substance so that an antibody labeled with the labelling substance can appropriately be prepared.

The detection means for lipocalin 15 is preferably the detection means for the protein, and more preferably the antibodies. Details of the antibodies are the same as those described above.

The test reagent of the present invention may comprise lipocalin 15, mRNA or a protein (preparation). Such a preparation can be used, for example, as a positive control, and/or for making a standard curve for quantification.

The test reagent of the present invention when comprises an antibody may further comprise components required for an immunoassay by antibodies. Such components include the labelling substance as described above and a substrate of an enzyme, and a diluent, a secondary antibody, a stabilizer for antibodies, a device (e.g., biopsy needle) or a solution (e.g., saline) for being able to take a sample from a mammal.

In certain embodiments, the test reagent of the present invention can be used as an intranasal endoscope test reagent. In such a case, the test reagent of the present invention preferably comprises an antibody labelled with a labelling substance, or comprises the antibody and the labelling substance separately so that the olfactory mucosa can be stained through lipocalin 15. Staining can be performed in any manner such as spray or application.

### EXAMPLES

Hereinafter, the present invention will be described in detail with reference to Examples, but the present invention is not limited the following Examples.

### (Example 1) Construction of expression vector for human LCN15

Primary sequence information of human LCN15 (hLCN15) was acquired from RSCB (https://www.rcsb.org/). A nucleotide excluding a region of a signal peptide, which corresponds to a mature protein, was synthesized at Eurofins (SEQ ID NO:1), and subcloned into a multicloning site of pET16b according to standard methods so that a His tag was genetically fused to an N-terminus of hLCN15 upon being expressed.

### (Example 2) Preparation of hLCN15 protein using Escherichia coli

BL21 (DE3) was transformed with the obtained plasmid by a heat shock method, inoculated onto an LB plate (containing 100 µg/L ampicillin) and statically cultured at 37°C for 13 hours. A formed colony was picked up, transferred to a 2 L flask containing 600 mL medium (MagicMedia, Thermo Fisher), cultured at 37°C for 16 hours with shaking at 200 rpm, and further cultured at 20°C for 20 hours. Microbial cells were collected by centrifugation at 6000 x g for 10 minutes, and stored in a freezer at - 80°C until disruption. In addition, a portion of the microbial cells were electrophoresed on 10 to 20% SDS-PAGE (Gellex International), and expression of His-hLCN15 was confirmed.

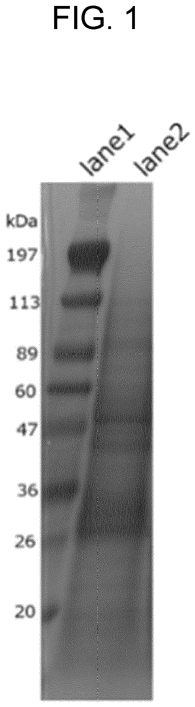

200 mL of the expressing microbial cells were lysed in lysis buffer (50 mM Tris-HCl, pH9, 1 M NaCl, 2 mM MgaCl₂, 2 mM CaCl₂, 4 mM DTT, 10% glycerol), then disrupted by ultrasonication (INSONATOR 201M, KUBOTA Corporation: 180W/30 min/4°C), subsequently centrifuged at 800 xg for 10 minutes, and then ultracentrifuged with AVANTI JXN-30 (Beckman) at 108,000 xg for 30 minutes. The resulting supernatant was applied onto a His-tag column that was an econopack column (Bio-Rad) with BV=1 mL, and the column was washed with the lysis buffer and further washed with 10 mL of PBS(-). Next, 5 mL of digestion buffer (50m M Tris-HCl, pH8.0, 100 mM NaCl, 5 mM CaCl₂) was added, 5 µL of Factor X (Merck) was added, and then the mixture was incubated at 37°C for 12 hours. Subsequently, a His supernatant was collected and concentrated by Amicon ultra MWCO10k (Merck). Its purity was analyzed by SDS-PAGE. No band derived from contaminated proteins was detected on the SDS-PAGE, a single band corresponding to hLCN15 was observed, and thus its purity is believed to be 90% or more (Fig. 1).

### (Example 3) Production of antiserum using hLCN15 as antigen

For an antibody, the antibody production service available from Eurofins Genomics was utilized. Specifically, rabbits were immunized with a prepared hLCN15 protein, i.e., 0.3 mg at the first immunization (Day 0), 0.2 mg at the second immunization (Day 28), 0.2 mg at the third immunization (Day 35) and 0.2 mg at the fourth immunization (Day 42). A blood sample was collected as a trial before the third immunization (Day 35) and an antibody titer was confirmed to sufficiently increase by ELISA. A whole blood sample was collected on Day 49, and antiserum (hLCN15 polyclonal antibody) was obtained.

### (Example 4) Collection of human olfactory mucus

Human olfactory mucus samples were collected by a noninvasive method. To collect an olfactory mucus sample, a subject got down on all fours and put a top of head on a floor so that a head region became inverse. 1 mL of warmed saline to 37°C was run into a one side of a nasal cavity, and retained in an olfactory region in an upper part of the nasal cavity. After being left standing for 10 minutes, the subject got up, and a liquid that ran out from the nasal cavity was collected into a tube. For a respiratory epithelial mucus sample, an absorptive sponge for surgery (Merocel (registered trademark), Medtronic Japan, Tokyo, Japan) was inserted into the nasal cavity, left standing for 10 minutes, and then collected.

### (Example 5) Western blotting method for human olfactory mucus using hLCN15 polyclonal antibody

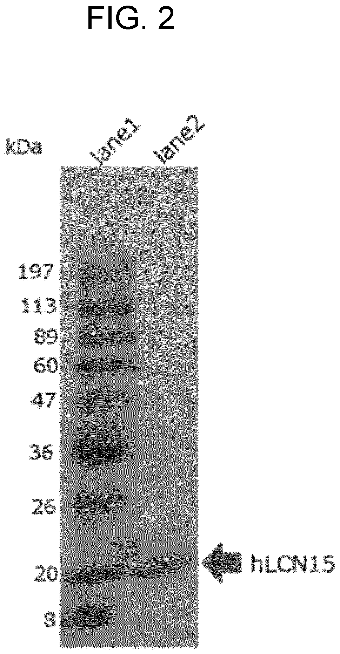

Each 7.5 µL of the olfactory mucus sample or the respiratory epithelial mucus sample was mixed with 4 x LDS sample buffer (Life Technologies), and 6 µL of each mixture was applied onto 10 to 20% SDS-PAGE (Gellex International). The mixture was electrophoresed at 200 V/80 mA for 35 minutes, and then transferred onto a PVDF membrane at 20 V/2 A for 7 minutes using iBlot system (Life Technologies). The resulting transferred membrane was subjected to an antigen antibody reaction using iBind system (Life Technologies). The hLCN15 polyclonal antibody and a goat anti-rabbit IgG antibody (KPL, #474-1506) were used as a primary antibody and a secondary antibody, respectively. Subsequently, a colorimetric reaction was performed using Super Signal (registered trademark) West Dura Trial Kit (Thermo Fisher), and data was scanned into a computer using Amwesham Imager 600 (Amersham). As a result, it was confirmed that the produced hLCN15 polyclonal antibody recognized the hLCN15 protein as the antigen (Fig. 2).

### (Example 6) Detection of hLCN15 in human olfactory mucus using hLCN15 polyclonal antibody

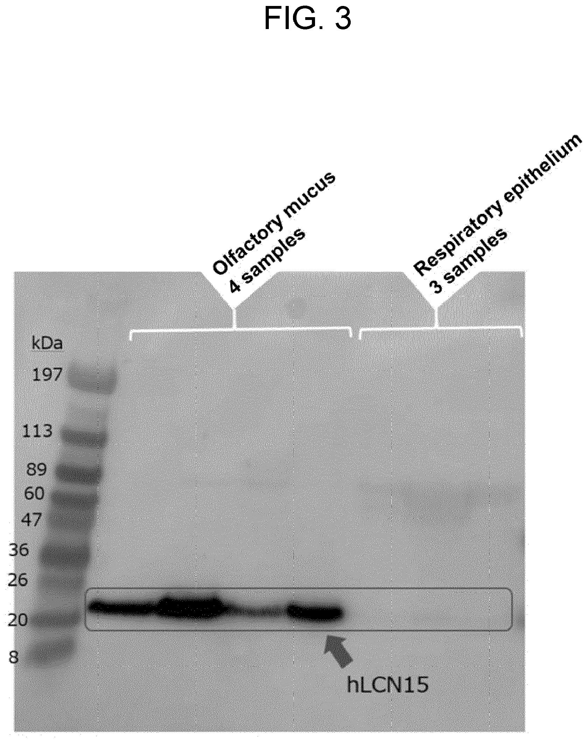

A western blotting method for the olfactory mucus samples and the respiratory epithelial mucus samples was performed using the prepared anti-hLCN15 polyclonal antibody. As a result, hLCN15 was below detection limit in all of the respiratory epithelial samples (n=3), but was detected in all of the olfactory mucus samples (n=4) (Fig. 3). The above data revealed that the prepared anti-hLCN15 polyclonal antibody was able to detect the hLCN15 protein in the human samples and that the hLCN15 protein was present in the olfactory mucus.

### (Example 7) Collection and fixation of human olfactory mucosa tissue

Human olfactory mucosa tissue samples were collected and then fixed in 10% neutral buffered formalin (Muto Pure Chemicals) at room temperature for a week. Subsequently, the samples containing bone tissue were decalcified in 10% ethylenediaminetetraacetic acid (EDTA) at room temperature for 4 weeks. Then, the samples were dehydrated by a series of alcohol, and then embedded with paraffin. Coronal sections having a thickness of 4 µm were prepared on slide glasses (Matsunami Glass Ind.) coated with MAS.

### (Example 8) Tissue immunostaining of human nasal sections using hLCN15 antiserum

### (1) Method

Prepared sections were deparaffinized and hydrophilized using a series of xylene/ethanol, and then antigen activation was performed using an antigen activation solution (DAKO, S1700) in an autoclave at 120°C for 20 minutes. Subsequently, endogenous peroxidase was blocked with methanol containing 3% hydrogen peroxide for 10 minutes. Non-specific antibody reactions were decreased by treating with a blocking solution (Nacalai Tesque, Blocking One Histo) at room temperature for 10 minutes, and then the section was reacted with the rabbit anti-hLCN15 antiserum (500 times dilution with an antibody diluent obtained by diluting the blocking solution 20 times with TBS-T), a rabbit anti-cytokeratin 18 (CK18) antibody (250 times dilution with the above antibody diluent), or a rabbit anti-PGP9.5 antibody (500 times dilution with the above antibody diluent), respectively at room temperature for one hour. After the reaction, the sections were washed with PBS, and reacted with an HRP-conjugated anti-rabbit IgG secondary antibody (Simple Stain MAX-PO (R), NICHIREI) at room temperature for 30 hours. A colorimetric reaction with HRP was performed using diaminobenzidine (DAB, NICHIREI). The sections were counterstained with hematoxylin, and then dehydrated and cleared.

### (2) Results

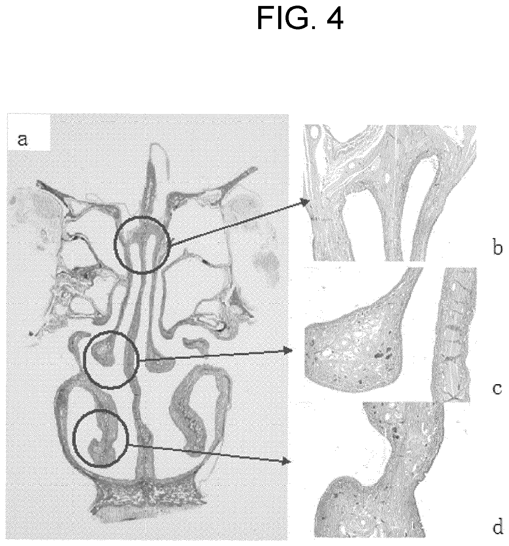

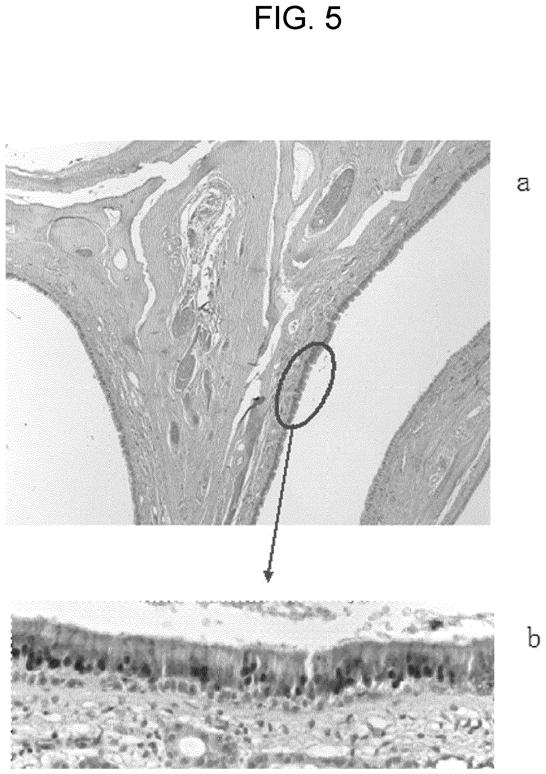

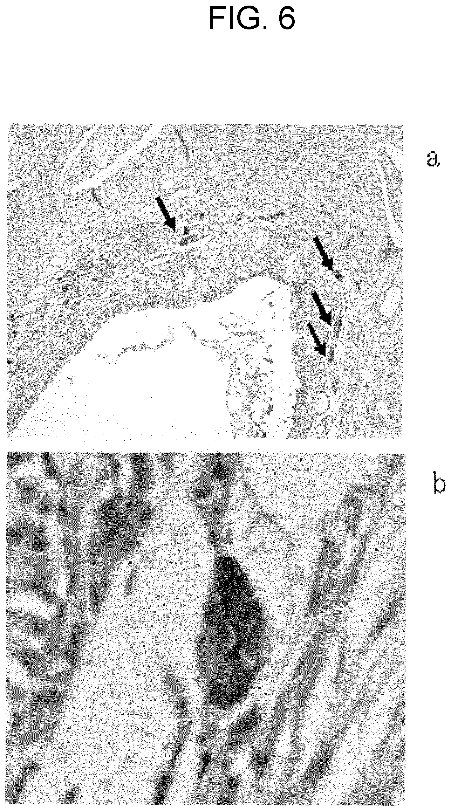

In the staining of a large section including the human entire nasal cavity, staining by the anti-hLCN15 antibody was not observed in a lower part of the nasal cavity and was observed in a lamina propria of an olfactory cleft that was an upper part of the nasal cavity (Fig. 4a to 4d). A portion stained with the anti-hLCN15 antibody is adjacent to a portion stained with the anti-PGP9.5 antibody (neural cell marker) and a portion stained with the anti-CK18 antibody (olfactory epithelium support cell marker), and thus appeared to be the olfactory mucosa (Fig. 5a, 5b). It was confirmed in the observation at high magnification that hLCN15 was expressed in glandular tissue thought to be Bowman's gland (Fig. 6a, 6b). That is, the expression of hLCN15 was significantly correlated with localization of the olfactory mucosa (Bowman's gland).

Differently from mice and rats, the olfactory mucosa has remarkably regressed and denatured in the human, and a region where olfactory nerve cells remain (PGP9.5 positive region) is confined. A CK18 positive region indicates that the support cells (not present in respiratory epithelium) that originally cover olfactory nerve epithelium remain. When the olfactory nerve epithelium is highly degenerated, the support cells are lost and it becomes similar to the respiratory epithelium. However, when the olfactory nerve epithelium moderately is degenerated, the olfactory nerve cells in an epithelial middle layer are lost, but the support cells remain (Reference: Child KM et al : The Neuroregenerative Capacity of Olfactory Stem Cells Is Not Limitless: Implications for Aging. J. Neurosci. 2018; 38: 6806-6824).

Therefore, states of the olfactory nerve epithelium are determined as follows, depending on a staining pattern of each cell marker.
PGP positive and CK18 positive region: no or mild degeneration of olfactory nerve epithelium;
PGP negative and CK18 positive region: moderate degeneration of olfactory nerve epithelium; and
PGP negative and CK18 negative: high degeneration of olfactory nerve epithelium.

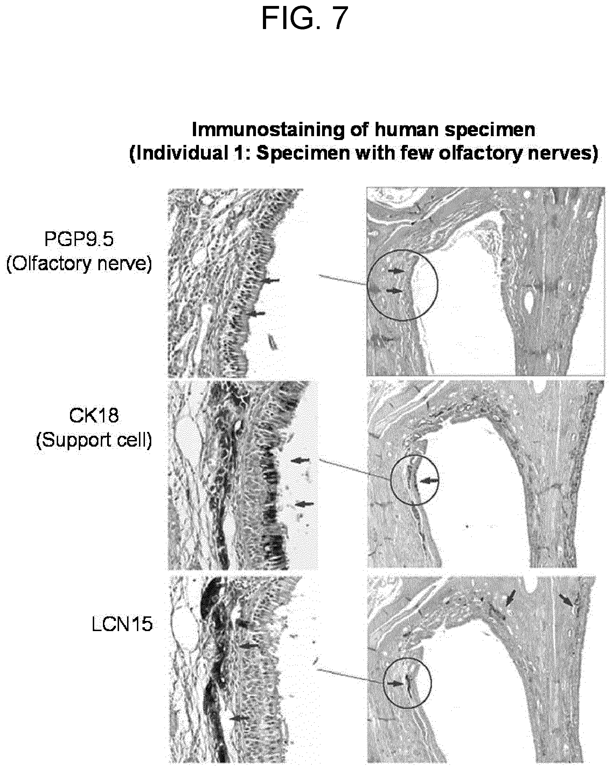

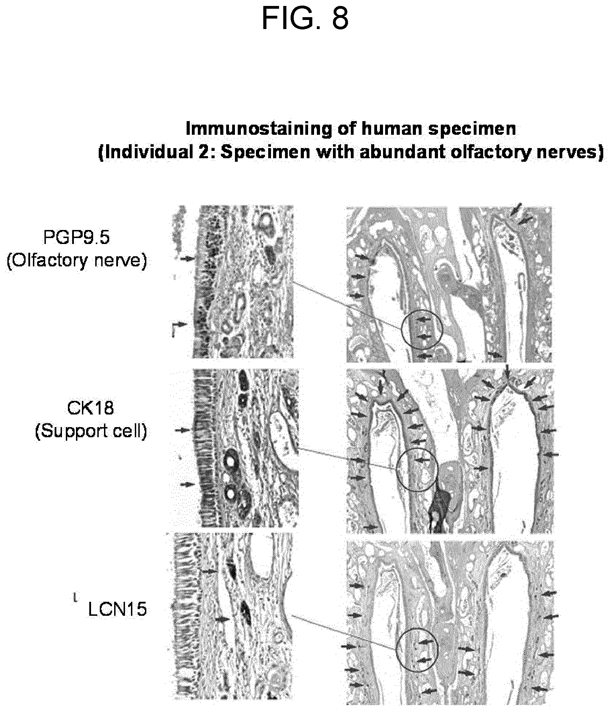

This time, the human nose large sections from two individuals were used for the tissue immunostaining. In the individual 1, the degeneration of the olfactory mucosa was slightly higher and PGP9.5 positive cells less remained. In the individual 2, slightly more nerves were observed to remain (Fig. 7 and Fig. 8). In consistency with this, areas stained with the anti-CK18 were in a narrow range in the individual 1 and more remained in the individual 2 (Fig. 7 and Fig. 8). Also, areas positively stained with LCN15 was confined in the individual 1 and distributed in a wider range in the individual 2 (Fig. 7 and Fig. 8). That is, the expression of LCN15 was significantly correlated with the function or the disorder associated with the olfactory mucosa.

Taken together, it was demonstrated that LCN15 can become an indicator of the olfactory mucosa, or the function or the disorder associated with the olfactory mucosa.

### (Example 9) Evaluation of hLCN15 in human olfactory mucus using hLCN15 polyclonal antibody

### (1) Western blotting of hLCN15 in human olfactory mucus using hLCN15 polyclonal antibody

Each 1.82 µg protein of human olfactory mucus or respiratory mucus was mixed with 4x LDS sample buffer (Life Technologies), and each 6 µL of the mixture was applied onto 10-20% SDS-PAGE (Gellex International). The mixture was electrophoresed at 20 V/80 mA for 35 minutes, and then transferred onto a PVDF membrane at 20 V/2 A for 7 minutes using iBlot system (Life Technologies). The resulting transferred membrane was subjected to an antigen antibody reaction using iBind system (Life Technologies). The hLCN15 polyclonal antibody and a goat anti-rabbit IgG antibody (KPL, #474-1506) were used as a primary antibody and a secondary antibody, respectively. Subsequently, a colorimetric reaction was performed using SuperSignal (registered trademark) West Dura Trial Kit (Thermo Fisher), and data was scanned into a computer using Amwesham Imager 600 (Amersham).

### (2) Quantification of hLCN15 in human olfactory mucus using hLCN15 polyclonal antibody

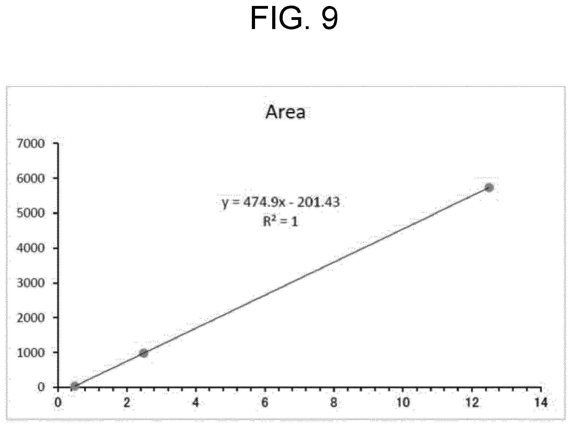

When the western blotting was performed, the hLCN15 protein at 12.5, 2.5 and 0.5 ng/lane was electrophoresed on the same gel, and subsequently transferred together onto a PDVF membrane. A densitogram was prepared from a 16 bit image data of chemiluminescence from detected hLCN15 bands to obtain an area under waveform, and a standard curve was prepared. Likewise, a densitogram was prepared from a 16 bit image data of chemiluminescence from detected hLCN15 bands in the samples to obtain the area under waveform, and amounts of the hLCN15 bands were converted using the standard curve. One example of the standard curve is shown in Fig. 9 (actually the standard curve was prepared per one gel/membrane).

### (3) Results

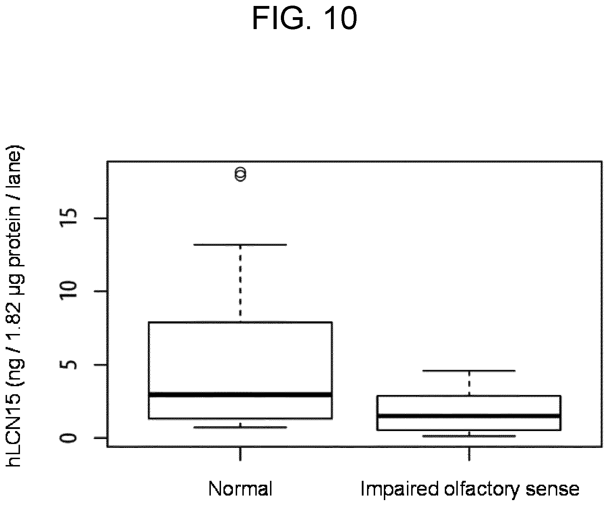

The subjects were divided into a normal olfactory sense group (subjects who were able to identify a threshold 2 in all of odorous substances A, B, C, D and E in the standard olfactometry) and an impaired olfactory sense group (subjects who were not able to discriminate the threshold 2 in any of odorous substances A, B, C, D and E in the same test). Amounts of LCN15 per 1.82 µg protein of the olfactory mucus were quantified and compared between the groups, and were significantly smaller in the impaired olfactory sense group compared to the normal olfactory sense group (Welch t test, p=0.009) (Table 1, Fig. 10).

**Table 1. Amounts of LCN15 (ng) per 1.82 µg protein of the olfactory mucus**

| | Normal olfactory sense | Impaired olfactory sense |
|---|---|---|
| 1 | 9.95 | 0.48 |
| 2 | 0.98 | 0.12 |
| 3 | 1.03 | 2.01 |
| 4 | 1.47 | 2.87 |
| 5 | 17.89 | 4.59 |
| 6 | 7.67 | 2.93 |
| 7 | 13.19 | 0.55 |
| 8 | 18.13 | 0.66 |
| 9 | 3.22 | 2.56 |
| 10 | 4.39 | 1.04 |
| 11 | 10.04 | |
| 12 | 5.00 | |
| 13 | 0.89 | |
| 14 | 2.95 | |
| 15 | 2.75 | |
| 16 | 3.42 | |
| 17 | 1.65 | |
| 18 | 1.22 | |
| 19 | 0.90 | |
| 20 | 8.12 | |
| 21 | 1.48 | |
| 22 | 0.72 | |
| 23 | 1.43 | |
| Average | 5.15 | 1.78 |

### (Example 10) Reporter assay using olfactory receptor-expressing cells

### (1) Materials

### (1-1) Cloning of human olfactory receptor gene

A human olfactory receptor gene was cloned by a PCR method using a human olfactory receptor gene purchased from TrueClone cDNA Clone Collection (OriGene) as a template base on sequence information registered in GenBank. Each gene amplified by the PCR method was subcloned into downstream of a Rho tag sequence of a Rho-pME18S vector (K. Kajiya et al., Journal of Neuroscience, 15 August 2001, 21 (16) 6018-6025) using EcoRI and XhoI sites to obtain an expression vector for the human olfactory receptor.

### (1-2) Production of expression vector for bat RTP1s

A bat RTP1s gene (nucleotide sequence from A at position 109 to a 3' terminus of GenBank Accession No. XM_006765851) was synthesized using the artificial gene synthesis service from Eurofins Genomics, and cloned into a pcDNA3.1(+) vector (Thermo Fisher Scientific) using HindIII and EcoRI sites to obtain an expression vector pcDNA3.1-microbat RTP1s for bat RTP1s.

### (1-3) Production of expression vector for human Golf

A full length cDNA sequence encoding a human Golf protein has been registered in GenBank at NCBI (GenBank accession No. NM_182978). RT-PCR was performed using human mRNA as a template and using primers of SEQ ID NOS:6 and 7. The amplified cDNA fragment was cloned into HindIII-EcoRI site of a plasmid pcDNA3.1 (+) (Life Technologies) using GENEART Seamless Cloning and Assembly Kit (A13288, Life Technologies) to obtain an expression vector pcDNA3.1-Golf for human Golf.

### (1-4) Production of expression vector for rat Ric8B

A full length cDNA sequence encoding a rat Ric8B protein has been registered in GenBank at NCBI (GenBank accession No. NM_175598). RT-PCR was performed using rat mRNA as a template and using primers of SEQ ID NOS:6 and 7. The amplified cDNA fragment was cloned into HindIII-XbaI site of a plasmid pcDNA3.1 (+) (Life Technologies) using GENEART Seamless Cloning and Assembly Kit (A13288, Life Technologies) to obtain an expression vector pcDNA3.1-Ric8B for rat Ric8B.

### (1-5) Production of olfactory receptor-expressing cells HEK293T cells that expressed the olfactory receptor

(OR X) was prepared by the following procedure. A mixed solution of the expression vector that had a composition shown in Table 2 was prepared, left in a clean bench for 20 minutes, and subsequently added to HEK293T cells (2.5 x 10⁶ cells/10 cm dish) seeded in a 10 cm dish on the previous day. The HEK293T cells were cultured in an incubator with 5% CO₂ at 37°C for 5 hours, and then 100 µL (2.5 x 10⁵ cells/mL) was seeded in each well of a 96 well plate (BD) and cultured overnight in the incubator with 5% CO₂ at 37°C

**Table 2. Composition of mixed solution for expression vector**

| | |
|---|---|
| OPTI-MEM (GIBCO) | 2.4ml |
| Expression vector for human olfactory receptor | 0.8µg |
| pGL4.29[luc2P/CRE/Hygro] Vector, Promega | 0.16µg |
| pGL4.74[hRluc/TK] Vector, Promega | 0.08µg |
| pcDNA3.1-microbat RTP1s | 0.16µg |
| pcDNA3.1-Golf | 0.08µg |
| pcDNA3.1-Ric8B | 0.08µg |
| Lipofectamine 2000 (Invitrogen) | 27.2µl |

### (2) Luciferase assay

The olfactory receptor (OR X) expressed on HEK293T cells is conjugated with Golf to activate adenylate cyclase, thereby increases an amount of intracellular cAMP. In this Example, the measurement for response of the olfactory receptor to a test substance employed luciferase reporter gene assay in which increase of the amount of intracellular cAMP was monitored as increase of a luminescence value derived from firefly luciferase. The "luciferase reporter gene assay" is also referred to as a "luciferase assay". Firefly luciferase is expressed from a firefly luciferase gene carried by pGL4.29 [luc2P/CRE/Hygro] vector in an intracellular cAMP amount-dependent manner. In conjugation with this, a luminescence value derived from Renilla luciferase was used as an internal standard for correcting errors of a gene transfer efficiency and a cell number in each well. Renilla luciferase is constitutively expressed from a Renilla luciferase gene carried by pGL4.74[hRluc/TK] vector under the control of CMV promoter.

### (3) Measurement for effect of concurrent addition of LCN15

HEK293 cells are allowed to express the olfactory receptor (OR X) and a luciferase assay is performed. The medium is removed from the culture obtained in (1-5) above, and 15 µL of CD293 medium, isovaleric acid (30 µM), and a mixed solution of isovaleric acid (30 µM) and LCN15 (1 µM) are added to use as a reaction solution. A solution of isovaleric acid (30 µM), and the mixed solution of isovaleric acid (30 µM) and LCN15 (1 µM) are prepared by dissolving them in the CD293 medium (Invitrogen). The cells are cultured in a CO₂ incubator at 37°C for 3 hours to allow to sufficiently express a firefly luciferase gene in the cells. A luminescence value derived from the intracellular firefly luciferase is measured to use as an "Luc value". A luminescence value derived from intracellular Renilla luciferase is measured to use as a "hRLuc value". The luminescence value derived from each luciferase is measured using Dual-Glo (trademark) Luciferase assay system (Promega) according to an operating manual for the product. The luminescence value derived from the firefly luciferase induced by stimulation with the test substance (Luc value) is divided by the luminescence value derived from the Renilla luciferase in the same well to obtain an "Luc/hRLuc value". The Luc/hRLuc value is used as an indicator of response intensity of the olfactory receptor (OR X) to the test substance.

As a result, the response intensity of the olfactory receptor in the mixed solution of isovaleric acid and LCN15 can be more enhanced as compared to the solution of isovaleric acid alone. That is, it is conceivable that LCN15 can be correlated with the function or the disorder associated with olfactory mucosa via the olfactory receptor (OR X) .

### INDUSTRIAL APPLICABILITY

As described above, the method and the reagent of the present invention are useful for the tests in the mammals such as humans.

## Claims

1. A test method for olfactory mucosa or olfactory mucus in a mammal, comprising measuring an amount of lipocalin 15 in an olfactory mucosa sample or an olfactory mucus sample taken from the mammal.

2. The test method according to claim 1, wherein the amount of lipocalin 15 as a protein is measured using an antibody against lipocalin 15.

3. The test method according to claim 1 or 2, wherein the amount of lipocalin 15 as a protein is measured in the olfactory mucus sample taken from the mammal.

4. The test method according to any one of claims 1 to 3, wherein the mammal is a human.

5. A test reagent for olfactory mucosa or olfactory mucus in a mammal, comprising a detection means for lipocalin 15.

6. The test reagent according to claim 5, wherein the detection means is an antibody.


### [Sequence Listing]

SEQUENCE LISTING

<110>  Ajinomoto Co., Inc.

<120>  Method of testing olfactory mucosa or olfactory mucus in mammal

<130>  AM-R011798

<150>  JP2018-200100
<151>  2018-10-24

<160>  1

<170>  PatentIn version 3.5

<210>  1
<211>  165
<212>  PRT
<213>  Homo sapiens

<400>  1

Met Glu Val Leu Leu Gln Pro Asp Phe Asn Ala Glu Lys Phe Ser Gly
1               5                   10                  15

Leu Trp Tyr Val Val Ser Met Ala Ser Asp Cys Arg Val Phe Leu Gly
            20                  25                  30

Lys Lys Asp His Leu Ser Met Ser Thr Arg Ala Ile Arg Pro Thr Glu
        35                  40                  45

Glu Gly Gly Leu His Val His Met Glu Phe Pro Gly Ala Asp Gly Cys
    50                  55                  60

Asn Gln Val Asp Ala Glu Tyr Leu Lys Val Gly Ser Glu Gly His Phe
65                  70                  75                  80

Arg Val Pro Ala Leu Gly Tyr Leu Asp Val Arg Ile Val Asp Thr Asp
                85                  90                  95

Tyr Ser Ser Phe Ala Val Leu Tyr Ile Tyr Lys Glu Leu Glu Gly Ala
            100                 105                 110

Leu Ser Thr Met Val Gln Leu Tyr Ser Arg Thr Gln Asp Val Ser Pro
        115                 120                 125

Gln Ala Leu Lys Ser Phe Gln Asp Phe Tyr Pro Thr Leu Gly Leu Pro
    130                 135                 140

Lys Asp Met Met Val Met Leu Pro Gln Ser Asp Ala Cys Asn Pro Glu
145                 150                 155                 160

Ser Lys Glu Ala Pro

                165